Europäisches Patentamt

European Patent Office

Office européen des brevets

(11) Publication number: **0 418 113 A2**

# EUROPEAN PATENT APPLICATION

(21) Application number: 90402409.8

(22) Date of filing: 31.08.90

(51) Int. Cl.5: **C12Q 1/18, C12Q 1/02**

(30) Priority: 11.09.89 IL 91596

(43) Date of publication of application:
20.03.91 Bulletin 91/12

(84) Designated Contracting States:
**BE DE ES FR GB IT NL**

(71) Applicant: ORGENICS Ltd.
**Industrial Zone**
**Yavne 70650(IL)**

(72) Inventor: **Reinhartz, Avraham**
**1 Rue Israel Shahar**
**Rehovot(IL)**
Inventor: **Aldadjem, Sarah**

**Kfar Hanagid(IL)**
Inventor: **Fish Falk**
**4 rue Dresner**
**Tel-Aviv(IL)**

(74) Representative: **Orès, Bernard et al**
**Cabinet ORES 6, Avenue de Messine**
**F-75008 Paris(FR)**

(54) **Microbiological assay kit and method for detecting antibacterial compounds.**

(57) This invention relates to a microbiological assay kit and method for detecting antibacterial compounds in a specimen, particularly useful for food and dairy industry.

Said method comprises the steps of :

a) admixing said specimen with viable bacteria exhibiting enhanced sensitivity to said antibacterial compound to form a mixture ;

b) incubating said mixture under optimal growth conditions;

c) diluting said mixture with an aqueous solution of signal generating reagents especially selected to detect at least one metabolic marker, existing in said bacteria;

d) incubating said mixture (c) for sufficient time for said signal to be detected;

e) measuring or detecting said signal in said mixture (c);

f) comparing said signal to a control specimen, free of said antibacterial compound, which was simultaneously subjected to said steps (a) to (e).

# MICROBIOLOGICAL ASSAY KIT AND METHOD FOR DETECTING ANTIBACTERIAL COMPOUNDS

This invention relates to a microbiological assay kit and method for detecting antibacterial compounds in a specimen, particularly useful for food and dairy industry.

Current detection systems for antimicrobial compounds fall into two major categories :

1) Specific assay methods based on specific receptors for the antibiotic in question. In these methods, a constant amount of labeled antiobiotic (usually radioactive) competes with the analyte in the milk specimen on a limited number of receptor sites. The advantage to such methods is in their speed. The main drawback is the specificity of each method to a single antibiotic. Thus, such methods will not detect antibiotics which they were not meant for. Another drawback is the use of radioactive tracers, which limited the operation of such assays to certified laboratories or enclosed automatic machinery.

2) Biological, Non-specific assay methods are based on the ability of antibiotics to inhibit metabolic processes of susceptible microorganisms. The metabolic activity can be demonstrated, for example, by the ability of the susceptible microorganism to produce acid, which changes the color of an acid/base indicator incorporated on the growth medium.

The main advantage of biological assays is their theoretical capability of detecting any antibiotic. The drawbacks involve lack of information on the nature of the detected antibiotic and the relatively long duration of the assay. The Delvotest® (commercially available from Gist Brocades NV, Delft, Holland) is an example of biological assay kit for antibiotic in milk. The detection of low concentration (<7 mIU/ml), of penicillin G by a biological assay requires a very long incubation period (2.5-3 hrs) of the specimen with the indicator organism. This is the outcome of the unique mechanism of action of all B-lactam antibiotics in general and Penicillin in particular. Those antibiotics inhibit cell wall synthesis, but they do not interfere at all with the bacterial cell metabolism. The antibiotic treated cells continue to metabolize and even multiply. Thereby, the progeny of antibiotic treated cells have decreasing amounts of cell wall. After some generations of growth in presence of the antibiotic the cell wall virtually disappears. Only at this stage the cell membrane can be affected and cellular metabolism is inhibited.

The presence of penicillin and other antibiotics in milk interfered with the processing of milk by the dairy industry. The antibiotics are introduced into the milk by microbiological fermentation systems (e.g. production of yogurt, cheese, sour cream, sour milk, etc) monitor each batch of delivered milk for the presence of antibiotics, with penicillin -as the major drug of interest, due to its wide use in veterinary medicine (cheap and effective).

The long duration of current biological assays for Penicillin presents operational difficulty to the dairy industry. Newly arrived milk shipments have to be stored separately until the slow biological test is completed. Only then, can the milk be transferred to further processing or alternatively be dispensed if it contains detectable antibiotic activity.

For example, a rapid biological assay for antibacterial activity in general and penicillin activity in particular will allow the dairy operation a wide spectrum test which can be performed on milk shipments before they are unloaded. If, in addition, the test will be sufficiently simple to use by non-professionals (e.g. drivers, farmers) it will allow a further decrease in milk processing duration by running the assay aboard the milk transporting vehicle, so that the test results are already known when the load arrives at the dairy. A simple rapid assay will be useful to the dairy farmer who will be able to determine residual antibiotic levels in milk of previously treated cows and from the results will be able to decide if the aforementioned milk can be used or, alternatively, be destroyed.

It is therefore clear that a rapid, sensitive biological assay will be highly desirable for the food industry and particularly dairy farming and industry.

It has been unexpectedly demonstrated by the inventors that antibacterial activity can be detected by a biological assay with a total duration of 30 minutes or less. The sensitivity of the assay to penicillin and other antibacterial compounds is comparable to or better than the commercially available tests. The assay procedure is simple and amenable to development in kit form.

The speed and sensitivity of the method are the result of an inventive combination and exploitation of the following principles :

(a) Generation of the highest possible signal from the biological system in question. This is achieved by ensuring that during the exposure of the bacteria to the specimen the best growth conditions are maintained and by selecting a metabolic marker which is found in abundance in the particular bacteria. For exemple, Streptococcus thermophilus is rich in lactate dehydrogenase, an enzyme detectable by its ability to reduce tetrazolium compounds and thereby generate a color. Dehydrogenase activity is a marker of the metabolic activity of the cell. The bacterium's natural growth medium is milk. Therefore, the combination of the excess enzyme, ideal growth in milk and the most sensitive commercially available tetrazolium dye will afford a high signal. High signal from a biological system can additionally be obtained by increasing the number bacterial cells.

(b) Sensitization of the bacteria to the antibacterial compound in question by subjecting them to stresses which relate to the mechanism of action of the antibacterial compound and/or by selecting a signal generating metabolic system which is affected by the compound. For example, Penicillin inhibits cell wall synthesis and increases cell membrane fragility. By subjecting the cell to hypertonic medium during exposure to Penicillin, followed by hypotonic shock during signal generation, even a limited cell wall and membrane damage will be amplified and will result in lysis and cell death. Also dehydrogenases are usually associated with membranes and require intact membrane structure for their activity. These enzymes will therefore be affected by limited membrane damage and afford high sensitivity to a Penicillin assay.

In one of the embodiments of the present invention the biological assay method is comprised of :

1. Cultivating a viable bacterial cell suspension in a suitable growth medium, preferably milk (-indicator strain). The bacterial are preferably gram positive and preferably of the species Streptococcus thermophilus (e.g. ATCC 14485) or Bacillus stearothermophilus (e.g. ATCC 10149).

2. Adding the bacterial cell suspension to the specimen, suspected of having an antibacterial compound and to a control specimen, known not to contain any antibacterial compound. A mixture of yeast extract and tryptose is preferably added to the specimen - indicator strain mixture, to get final concentrations between 0.1 and 0.4% each, preferably 0.25 % each.

3. Incubating the specimen, bacteria, yeast extract, tryptose mix for a period of up to 30 minutes, preferably 10-25 minutes, at the optimal temperature and aeration conditions for the indicator strain in question (e.g. 42C without shaking for Strep. thermophilus ; 55C with shaking for B. stearothermophilus ).

4. At the end of step (3), diluting (1:1-1:10) the mixture with an aqueous solution of 0.5 % glucose and a tetrazolium salt, preferably MTT (3-[4,5-Dimethylthiazol-2-yl]-2,5 diphenyltetrazolium bromide) at concentrations of 0.035 - 0.350 mg/mL, preferably 0.175 mg/mL. Alternatively, an aqueous solution of 0.5 % glucose and a pH-indicator preferably Bromocresol purple or Bromothymol blue can be used. The diluted mixture is incubated for 1 - 10 (preferably 5) minutes. In yet another alternative, diluting the mixture 1:1 - 1:10 in water and determining ATP (Adenosine Tri Phosphate) inside or outside the bacterial cells e.g. by any commercially available ATP quantitation reagent kit. Such reagent kits are available from Sigma Chemical Company, St Louis, MO, USA or Lumac BV, Landgraaf, Holland. Other signal generation systems, indicating viability, metabolic (anabolic or catabolic) activity or membrane integrity may be employed instead with a variable degree of sensitivity, ease of use and speed.

5. Comparing the result obtained in step 4. of the control suspension with that of the test specimen. In case of the tetrazolium dye, a lighter color of the specimen indicates antibiotic activity. In case of a pH indicator, different color indicates antibiotic activity. In the case of ATP quantitation, different level of ATP indicates antibiotic activity.

In another embodiment of the invention, the cultivation of the indicator bacterial strain (step (1) above) is accomplished in a medium made hypertonic by addition of e.g. sucrose to a final concentration of 0.6 - 1.2M, preferably 0.8M. In further embodiment of the invention, the assay medium is made hypertonic, e.g. by adding sucrose of the specimens and control to a final concentration of 0.6 - 1.2M, preferably 0.8M. In yet another embodiment the bacteria are freeze dried after cultivation (step 1) and rehydrated before use.

In another embodiment of the invention, the resultant color of the bacterial suspension (step 5) can be extracted with an organic solvent from the fluorocarbon group, preferably Arklone-pR (ICI), or by another organic solvent, such as 1-Butanol. In the case of the Freon extraction, the color concentrates in the interface between the solvent and aqueous phases after centrifugation. In the case of butanol, the color is concentrated in solvent phase, which is located on top of the aqueous phase following a short centrifugation (1-2 min). The color density of the butanol phase can be determined by a transmission spectrophotometer set at 570 nm.

The invention allows also for a reagent kit intended for the detection of antibacterial compounds in various specimens, preferably milk. In one embodiment, the kit will include a bacterial suspension, reagents for the generation of the signal (e.g. chromogenic enzyme substrate, pH indicator, luminescence system for detection of ATP). In a preferred embodiment of the kit, the bacteria are freeze dried and the signal generating reagents are supplied in a dry form, thus allowing long term storage of the kit. The kit may include also vials, test tubes and volumetric equipment.

The metabolic activity of bacteria is a commonly employed indicator for toxic and/or antibiotic activities. It is used to monitor toxic material in waste treatment systems (Anderson et al : "Comparison of microbial assay techniques for evaluating toxicity of organics in industrial wastes", in : Toxicity Screening Procedures

3

Using Bacterial Systems, Liu and Dutka, eds., Marcel Dekker, N.Y. 1984, pp. 215-232 ; Reinhartz, US Pat. 4,774,173 ; Bulich and Isenberg, Adv. Instrum. 35:35-40, 1980). Reinhartz (US Pat. 4,774,173) demonstrated that an increase in sensitivity to a wide variety of toxicants is attainable by subjecting the indicator bacteria to one or more stress treatments before exposing them to the toxican in question. Freeze drying and hypertonic shock were identified as effective stress treatments.

Metabolic activity of indicator bacteria can be measured or monitored by a variety of simple procedures, most notably change of pH in the growth medium or reduction of tetrazolium salts (Sacks, US Pat. 2,967,132) the last being an indicator of dehydrogenase activity in bacterial cells. Recently, the activity of a de novo synthesized enzyme, having a chromogenic substrate, has been employed as a measure of metabolic activity (Quillardet et al. Proc. Natl. Acad. Sci. USA, 79, 5971-5975), offering greater theoretical sensitivity and speed. Reinhartz (US Pat. 4,774,173) employed a de novo synthesized enzyme (B-galactosidase) as a measure of metabolic activity with increased sensitivity to a variety of toxicants and antibiotics.

In the current invention gram positive indicator bacterial strains can be employed due to the high sensitivity of such bacteria to penicillin and other B-lactam antibiotic drugs. Reduction of tetrazolium dye (particularly MTT) or pH change were chosen as measures of metabolic activity. The required sensitivity to penicillin in a short assay duration is attained by subjecting the indicator bacteria to hypotonic shock after incubation with the antibiotic. Cells in which cell wall synthesis was inhibited by the antibiotic, will be more fragile and will suffer membrane damage or complete lysis by the hypotonic shock. The metabolism in such cells is terminated whereas it is unaffected by the hypotonic shock in cells, in which cell wall synthesis was not pertubed by exposure to the antibiotic. Using hypotonic shock the toxic effect of penicillin and other B-lactam antibiotics can be demonstrated at an early stage of their interaction with the bacterial cell.

By way of exemples the embodiments of the invention are described as follows :

### Example 1

Cultivation of Streptococcus thermophilus for antibiotic assay .

(a) Streak Streptococcus thermophilus (ATCC 14485) suspension on solid medium, comprising 10 % Difco skim milk, 0,5 % Difco yeast extract, 2 % Agar in distilled water. Distinct colonies should emerge after overnight incubation at 42° C.

(b) Suspended a colony in 10 mL of a liquid medium, comprising 10 % Difco skim milk and 0.5 % Difco yeast extract in water (medium A). Incubate the suspension overnight at 42° C in 12 mL plastic, disposable centrifuge test tubes.

(c) Dilute the resultant suspension 1:10 into medium A and incubate at 42° C until the pH of the medium drops to 5.0 (about 2hrs). The resulting viable cell concentration is $1\text{-}2\times10^8/1\text{mL}$.

(d) Cell concentration can be increased by adding 1N NaOH solution until the pH is raised to 6.2, and continuing incubation at 42° C until the pH drops again to 5.0. This cycle can be repeated 5-7 times until the culture becomes stationary at $1\text{-}2\times10^9$ cells/mL.

(e) Adjust cell concentration to $1\times10^8$ cells mL and store at 6-10° C for up to 24 hrs for use in antibiotic assay. Alternatively, distribute 2 mL aliquots of the suspension into 12 mL Penicillin vials (24 x 45 mm), freeze in liquid nitrogen and dry on refrigerated shelves of a stoppering, programmable lyophilizer, employing a 48-72 hour program.

### Example 2

Detection of Penicillin in milk employing freshly cultivated or freeze-dried Strep. thermophilus cell suspensions .

(a) Add 2 mL of distilled water to each vial of dried Strep. thermophilus cells, prepared as described in Example 1. Freshly grown Strep. thermophilus at $1\times10^8$ cells/mL are used without any treatment.

(b) Construct reaction mixtures for the detection of antibiotic activity in milk specimens from 0.4 mL cell suspension (fresh or rehydrated lyophilized), 1.6 mL milk specimen, 0.05 mL 10 % Difco Yeast Extract and 0.05 mL 10 % Difco Tryptose. Include at least one control milk specimen, known not to contain any antibiotic drug, whenever the Antibiotic assay is employed.

(c) Incubate the complete antibiotic exposure mixtures of the control and unknown milk specimens for 25 minutes at 42° C.

(d) Upon completion of the incubation mix 0.4 mL aliquots of all reaction mixtures with 1.6 mL of 0.5 % glucose, 0.175 mg/mL MTT (3-[4,5-Dimethylthiazol-2-yl]-2.5 diphenyltetrazolium bromide) in water, and incubate for 5 minutes. The amount of color resulting from MTT reduction can be estimated by one of the methods detailed in Example 3.

Table 1 below presents the results obtained when testing a milk specimen, containing 7 mIU/mL of Penicillin G and measuring the amount of color, employing the butanol extraction method (detailed in Example 3).

Table 1

| Detection of Penicillin G in milk, employing fresh and lyophilized Strep. thermophilus cells. | | | |
|---|---|---|---|
| | OD 570 nm in | | |
| Cell Type | Control Specimen | 7 mIU/mL Penicillin Specimen | % Inhibition |
| Fresh | 0.195 | 0.088 | 55 |
| Dried | 0.156 | 0.062 | 60 |

It is clear that dried bacteria restore most of their dehydrogenase activity following rehydration and they exhibit a slightly higher sensitivity to 7 mIU/mL penicillin G than freshly grown cells.

Example 3

Optional methods for the measurement of reduced MTT dye formed in milk

The reaction mixture in the milk antibiotic assay is opaque, due to the presence of milk. In addition, the reduced formazan dye tends to precipitate out of aqueous solution, thus interfering with photometric determination. Various methods were developed for visual or instrumental determination of the reduced formazan dye.

(a) Direct visual analysis.

The blue-red reduced MTT dye is visible by direct examination of the reaction mixture. An antibiotic treated specimen will have less color than the control.

(b) Collection of dye by filtration.

The whole reaction mixture is filtered through glass filter paper circles (e.g. Whatman GF/F, 2.5 cm diameter). The color can be estimated visually or measured by a reflecting densitometer, such as Gretag D182 (Gretag Ltd., CH-8105 Regensdorf, Switzerland).

(c) Partial clarification of reaction mixture by solvent/detergent treatment.

To a 2 mL reaction mixture, add 4 mL of 50 % 1-Butanol, 10 % SDS in water. Following a 30 second vigourous agitation on a vortex mixer the suspension is partially clarified. Determine the color density by a spectrophotometer adjusted to 570 nm.

(d) Butanol extraction of MTT dye.

Transfer a 0.75 mL aliquot of the reaction mixture to a 2 mL microcentrifuge test tube. Add 0.75 mL 1-Butanol. Agitate on a vortex mixer for 15 seconds. Centrifuge the suspension at 10,000 x g for 2.5 minutes. Transfer 0.6 mL the upper clear, colored phase to a clean microcentrifuge test tube, containing 0.6 mL 1-Butanol. Agitate on a vortex mixer for 15 seconds. Centrifuge the suspension at 10,000 x g for 2.5 minutes. Transfer 1 mL the upper clear, colored phase to a clean cuvette. Determine the color density at 570 nm.

This method is highly reproducible and sensitive.

(e) Fluorocarbon extraction of MTT dye.

To a 2 mL reaction mixture add 0.5 mL fluorocarbon 113 (Arklon-P from ICI). Agitate vigorously for 15 seconds on a vortex mixer. The MTT dye is concentrated in the bottom phase, thus affording increased sensitivity and clarity of color. The color is estimated visually. Further concentration of the color can be attained by centrifuging the fluorocarbon specimen mixture for 10 seconds in a microcentrifuge (e.g. Eppendorf microfuge). The MTT dye concentrates in the interface between the aqueous and fluorocarbon phases.

Example 4

Demonstration of the importance of hypotonic medium during the MTT dye development

Milk specimens containing 3.5 or 7 mIU/mL of Penicillin G were tested with lyophilized Strep. thermophilus organisms, as described in Example 2. Color development for each antibiotic concentration was carried out either with the MTT solution as described in section (d) of Example 2, which is hypotonic or with the same solution, made isotonic by the addition of sucrose to 0.3M final concentration.

The amount of MTT color produced was determined by measuring the optical density of butanol extract made from the reaction mixtures (example 3 (d)). The results are presented in Table 2.

Table 2

| Effect of hypotonic shock during MTT color development on sensitivity to Penicillin G | | | |
|---|---|---|---|
| MTT solution | Penicillin concentration | OD at 570 nm | % inhibition |
| Isotonic | 0 | 0.117 | 0 |
| | 4 | 0.113 | 3 |
| | 7 | 0.077 | 34 |
| Hypotonic | 0 | 0.118 | 0 |
| | 4 | 0.052 | 56 |
| | 7 | 0.031 | 74 |

The results demonstrate that the hypotonic MTT dye solution enhances the inhibitory effect of Penicillin, probably by the mechanism discussed in the "Technological Background of the Invention" section.

6

## Example 5

### Sensitivity of milk penicillin assay method, employing lyophilized bacteria

Milk specimens containing 1-14 mIU:mL Penicillin G, were tested as described in Example 2 for lyophilized bacteria. Resulting color densities were determined as described in Example 3(d) and summarized in Table 3.

Table 3

| Sensitivity of Penicillin G detection using lyophilized Strep. thermophilus | | |
|---|---|---|
| Penicillin Concentration (mIU/mL) | OD at 570 nm | Inhibition % |
| 0 | 0.118 | 0 |
| 1 | 0.098 | 17 |
| 2 | 0.080 | 32 |
| 4 | 0.052 | 56 |
| 7 | 0.031 | 74 |
| 14 | 0.024 | 80 |

As low as 1 mIU/mL concentration of Penicillin G is detectable in milk, using the invention.

## Example 6

### Effect of incubation durations on the sensitivity of the Penicillin Assay

Milk specimens, containing 7 mIU/mL of Penicillin G were tested as described in Example 2 for lyophilized Strep. thermophilus cultivated to high density by pH adjustments. Incubation times of the milk specimens with the indicator bacteria varied between 10 and 25 minutes. Incubation with the MTT solution varied between 5 and 10 minutes. The amount of MTT color produced was determined by measuring the optical density of butanol extract made from the reaction mixtures (example 3 (d)). The resulting inhibition values for the various combinations are summarized in Table 4.

Table 4

| Effect of assay duration on the Inhibitory Effect of Penicillin G | | | |
|---|---|---|---|
| Duration of First Incubation (min) | Duration of Incubation with MTT (min) | Total Assay Duration (min) | Inhibition (%) |
| 10 | 5 | 15 | 7 |
| 15 | 5 | 20 | 21 |
| 20 | 5 | 25 | 77 |
| 25 | 5 | 30 | 62 |
| 10 | 10 | 20 | 42 |
| 15 | 10 | 25 | 42 |
| 20 | 10 | 30 | 53 |
| 25 | 10 | 35 | 57 |

The conclusion from the data presented in Table 4 is that for detection of 7 mIU/mL Penicillin G the total assay duration could be shortened to 20 minutes, by exposing the indicator bacteria to the milk specimen for 10 minutes and allowing color development to proceed for 10 minutes. The resulting 40 % inhibition is sufficiently high to allow reproducible detection of penicillin at 7 mIU/mL or above.

Example 7

Visual detection of antibiotics in milk employing lyophilized bacteria subjected to osmotic changes .

(a) Add 2 mL of a solution containing 2.5 % tryptose and 2.5 % yeast extract to a vial of dried Strep. thermophilus cells, cultivated to yield a high cell concentration (Example 1.d). Keep cold until use.
(b) To each 1 mL milk specimen or controls add 0.053 gr glucose and 0.1 mL of rehydrated bacterial suspension obtained in (a) above. Mix and incubate at 42° C for 25 min.
(c) Add 2 mL of a chromogenic mixture, containing 0.5 % glucose and 0.175 mg/mL MTT in water to each of the specimens and controls. Mix and incubate for 5 min at 42° C.
(d) Visually examine the color of each specimen and compare it to the color of the control. A color density lower than the color of the control indicates antibiotic activity in the specimen.
Table 5 below summarizes the sensitivity of the visual assay for various antibiotic drugs, added to antibiotic free milk specimens.

Table 5

| Sensitivity of the visual antibiotic assay | |
|---|---|
| Antibiotic | Sensitivity[a] |
| Penicillin G | 3.5 mIU/mL |
| Ampicillin | 2 ng/mL |
| Cloxacillin | 2 ng/mL |
| Chloramphenicol | 2.5 ug/mL |
| Tetracycline | 0.2 ug/mL |
| Oxytetracycline | 0.4 ug/mL |
| Polymyxin | 10 ug/mL |
| Sulfadiazine[b] | 20 ug/mL[b] |

[a] lowest concentration causing visually detectable decrease in color.

[b] test conducted in presence of 10 ug.mL trimethoprim, added to the milk specimen.

Example 8

Detection of Penicillin G in milk by Bacilus stearothermophilus

(a) Inoculate B. stearothermophilus ATCC 10149 into a liquid medium constructed from 10 % Difco skim milk and 0.5 % Difco yeast extract in water. Incubate at 55° C with constant (shaking) for 18 hrs.

(b) Dilute the overnight culture 1:20 into fresh liquid medium and continue shaking at 55° C for 2 hrs.

(c) Dilute 1:10 into liquid medium fortified with 0.8M sucrose and continue shaking at 55° C for 2.5 hrs. (The importance of the sucrose for sensitivity will be demonstrated in Example 8). Use immediately or keep on ice until ready to continue.

(d) Mix each Penicillin containing milk specimen with an equal volume of B. stearothermophilus suspension, as obtained in Step (c). Shake for 30 minutes at 55° C.

(e) Add MTT solution, as described in Example 2 (d) and continue shaking for 10 minutes at 55° C.

(f) Determine the amount of MTT dye as described in Example 3(b).

Results of an experiment are presented in Table 6.

Table 6

| Detection of Penicillin G in milk with B. stearothermophilus cells | | |
|---|---|---|
| Penicillin G Concentration (mIU/mL) | OD at 570nm | % Inhibition |
| 0 | 0.380 | - |
| 16 | 0.250 | 34 |
| 32 | 0.155 | 59 |

Example 9

Enhancement of B. stearothermophilus sensitivity of Penicillin by pre-incubation in hypertonic medium

Logarithmic B. stearothermophilus (ATCC 10149) cells were suspended in Difco Tryptic Soy broth with or without 0.8M sucrose, and incubated at 55°C with shaking for 1 hr. Aliquots of the bacterial suspension were mixed with equal volumes of Tryptic Soy broth specimens containing either 40 mIU/mL Penicillin G or 1 ug/mL tetracycline. Control reaction mixture was constructed with bacterial suspension and Tryptic Soy broth. Following a 30 min incubation at 55°C, all reaction mixtures were diluted 1:5 into 175 mg/mL MTT, 0.5 glucose in water and incubation continued for 5 minutes. The color suspension was clarified by vigorously mixing 0.5 mL aliquots with 0.7 mL isoamyl alcohol containing 0.04N hydrochloric acid. The optical density of the clarified mixtures was measured at 570 nm and the inhibition of color formation, relative to the control was calculated and is presented in Table 7.

Table 7

| Effect of pre-incubation in hypertonic medium on sensitivity of B. stearothermophilus to Penicillin | | |
|---|---|---|
| Pre-incubation Medium | % Inhibition with | |
| | Penicillin G | Tetracycline |
| Isotonic | 10 | 45 |
| Hypertonic | 54 | 40 |

It was shown that pre-incubation in hypertonic medium increases bacterial susceptibility to the toxic effect of Penicillin. The pre-incubation has no effect on the sensitivity to Tetracycline - an antibiotic which does not interface with cell wall synthesis.

Finally, although the invention has been specifically described with reference to particular means and embodiments it is to be understood that the invention is not limited to the particulars disclosed and extends to all equivalents within the scope of the claims.

## Claims

1. A method of assaying or detecting the presence and/or extent of an antibacterial compound in a specimen, comprising the steps of :
   (a) admixing said specimen with viable bacteria exhibiting enhanced sensitivity to said antibacterial compound to form a mixture ;
   (b) incubating said mixture under optimal growth conditions ;
   (c) diluting said mixture with an aqueous solution of signal generating reagents, especially selected to detect at least one metabolic marker, existing in said bacteria ;
   (d) incubating said mixture (c) for sufficient time for said signal to be detected ;
   (e) measuring or detecting said signal in said mixture (c) ;
   (f) comparing said signal to a control specimen, free of said antibacterial compound, which was simultaneously subjected to said steps (a) to (e).

2. The method as defined by claim 1 wherein said antibacterial compound is a toxicant or an antibiotic.

3. The method as defined by claim 1 wherein said bacteria are Gram positive.

4. The method as defined by claim 1 wherein said bacteria are selected from the group consisting of genus Bacillus .

5. The method as defined by claim 1 wherein said bacteria are selected from the group consisting of species Bacillus stearothermophilus .

6. The method as defined by claim 1 wherein said bacteria are selected from the group consisting of genus Streptococcus .

7. The method as defined by claim 1 wherein said bacteria are selected from the group consisting of species Streptococcus thermophilus .

8. The method as defined by claim 1 wherein said enhanced sensitivity was obtained by stressing said bacteria.

9. The method as defined by claim 8 wherein said stressing was obtained by increasing osmotic pressure of the said bacteria.

10. The method as defined by claim 9 wherein said stressing was obtained by adding sufficient amount of sucrose.

11. The method as defined by claim 1 wherein said enhanced sensitivity resulted from freeze drying of said bacteria.

12. The method as defined by claim 1 wherein said optimal growth conditions comprises addition of nutrients.

13. The method as defined by claim 12 wherein said nutrients are yeast extract and/or tryptose.

14. The method as defined by claim 1 wherein said signal generating reagents comprise chromogenic compound.

15. The method as defined by claim 14 wherein said chromogenic compound is a tetrazolium salt.

16. The method as defined by claim 1 wherein said signal generating reagents comprise pH indicator.

17. The method as defined by claims 15 and 16 wherein glucose is added.

18. The method as defined by claim 1 wherein in step (c) said mixture is further diluted with water and said detection in step (e) is made by quantitation of adenosine tri phosphate.

19. The method as defined by claim 1 wherein said detection is made by a densitometer or photometer.

20. The method as defined by claim 1 wherein said detection is made by solvent extraction.

21. The method as defined by claim 20 wherein said solvent is butanol or fluorocarbon.

22. A method of preparing indicator bacteria for use in detection the presence or extent of an antibacterial compound in a specimen comprising :

    (a) selecting bacteria for which said specimen constitutes the most advantageous growth medium

    (b) selecting a metabolic marker which is abundant in said indicator bacteria

    (c) subjecting said indicator bacteria to stress which enhances their sensitivity to said antibacterial compounds.

23. A method of assaying or detecting the presence and/or extent of an antibiotic in milk comprising the steps of:

    (a) admixing said milk with Streptococcus thermophilus bacteria to form a mixture ;

    (b) incubating said mixture under optimal growth conditions by adding yeast extract and tryptose in final concentration between 0.1 - 0.4 % ;

    (c) diluting said mixture (b) with an aqueous solution of 0.5 % glucose and a tetrazolium salt ;

    (d) incubating said mixture (c) for sufficient time; and

    (e) detecting the resultant color and comparing to a control specimen free of antibiotic wherein said control specimen was simultaneously subjected to said steps (a) to (d).

24. A kit for use in assaying or detecting the presence and/or extent of antibacterial compound in a specimen which comprises :

    (a) viable bacteria ;

    (b) reagents for enhancing sensitivity of said bacteria for said antibacterial compound ;

    (c) growth promoting medium for said bacteria;

    (d) reagents for signal generation.

25. A kit defined by claim 24 wherein a control specimen free of said antibacterial compound is added.

26. A mixture comprising a viable bacteria having enhanced sensitivity to an antibacterial compound and growth promoting and signal generating reagents.

27. A mixture of claim 25 further comprising a specimen and control specimen being assayed for the presence of an antibacterial compound.